# EUROPEAN PATENT APPLICATION

(11) **EP 3 831 418 A1**
(43) Date of publication of application: **09.06.2021**
(21) Application number: 19844468.9
(22) Date of filing: 24.07.2019
(51) Int. Cl.: A61L 27/14, A61L 27/54, A61L 27/58

(54) **BIOVEIL FOR THE TREATMENT OF WOUNDS AND BURNS**

(30) Priority: 01.08.2018 ES 201831222 U
(71) Applicant: Asociación de Investigación de la Industria Textil (AITEX), 03801 Alcoy (Alicante) (ES)
(72) Inventor: CAMBRA SÁNCHEZ, Vicente, 03801 Alcoy (ALICANTE) (ES); BLANES COMPANY, María, 03801 Alcoy (ALICANTE) (ES); MARCO BARRACHINA, Bruno, 03801 Alcoy (ALICANTE) (ES); PASCUAL MIRÓ, Carlos, 03801 Alcoy (ALICANTE) (ES); ORALES MARTÍN, Iñigo, 03801 Alcoy (ALICANTE) (ES); SEPÚLVEDA SANCHIS, Pilar, 46026 Valencia (ES); CASTELLANO IZQUIERDO, Delia, 46005 Valencia (ES); PÉREZ DEL CAZ, María Dolores, 46008 Valencia (ES)
(74) Representative: Ibarra Garcia, Isabel
(86) International application number: PCT/ES2019/070518
(87) International publication number: WO 2020/025843

(57) **Abstract**

The present invention relates to a bioveil for the treatment of wounds and burns, which is formed by a sheet of tangled polymer nanofibres of 50:50 esterified poly(D,L-lactic-co-glycolic) acid. The nanofibres have a nanometric diameter between 500 and 1500 nm and are disposed randomly, and the sheet has a grammage between 5 and 10 g/m² and is porous, hydrophilic and absorbable. The use of the bioveil in patients with second- and third-degree burns promotes the improvement of the quality of the healed tissue in terms of elasticity and traction and better skin graft take, promoting cell anchorage and blood vessel generation. The bioveil of the invention can be completely absorbed 90 days after its implantation.

## Description

### OBJECT OF THE INVENTION

The present invention relates to a bioveil, more particularly to a sheet the structure of which mimics the extracellular matrix of the native tissue of a patient and which, when applied on wounds or burns, provides a temporary scaffold enabling better cell anchorage, new dermal tissue formation, as well as greater angiogenesis.

The bioveil of the present invention is thereby formed by tangled polymer nanofibres of 50:50 esterified poly(D,L-lactic-co-glycolic) acid disposed randomly for the purpose of providing a porous, hydrophilic and absorbable sheet which, advantageously, will degrade completely 90 days after its placement on the wound or burn to be treated.

### BACKGROUND OF THE INVENTION

Biomaterials used during new tissue formation or for the treatment of wounds for the purpose of promoting cell anchorage and for the intention of providing a treated surface with a contraction-free tissue-like appearance are well-known in the prior art.

Poly(L-lactic) acid (PLLA), poly(D/L-lactic) acid (PDLLA) and poly (lactic-co-glycolic) acid (PLGA) have been known for a long time as degradable materials for implants, and they are all approved by the United States Food and Drug Administration (FDA) for such purpose. In fact, these elements have been used as tissue matrix support for bone, cartilage, liver, skin, urethra, bowel, tendons, and cardiovascular tissues.

In many cases, the biomaterials that are used do not present complete biodegradability, so it is necessary for the sheet of biomaterial that is placed to be removed after several days of action on the wound. In these cases, the removal of the bioveil placed on the wound involves the simultaneous elimination of those cell anchorages that may have been generated, so these biomaterials do not offer advantageous properties.

Additionally, another drawback of known biomaterials resides in the contraction they generate on the tissue surface of the treated wound. Therefore, in many cases, known biomaterials provide rapid wound healing but generate a wrinkled and anti-aesthetic surface, which is largely rejected by the patient not only because of its negative aesthetics, but also because of the lack of sensitivity and elasticity of the treated area.

Patent document number ES2338820T3 discloses a biodegradable, porous polymer material comprising poly(lactic-co-glycolic) acid intended for use in wound healing or regeneration processes. However, the document does not disclose that it relates to a product for prolonged use, that it can be completely absorbed, and that it provides better resulting scar quality.

The invention proposed in present specification provides a bioveil the structure of which solves the drawbacks described above, as it provides a bioabsorbable sheet which completely degrades and promotes angiogenesis and cell culture take, with less contraction of the treated surface.

### DESCRIPTION OF THE INVENTION

The proposed bioveil solves in a completely satisfactory manner the drawbacks set forth above, where its structure is formed by a sheet comprising tangled polymer nanofibres of 50:50 esterified poly(D,L-lactic-co-glycolic) acid, hereinafter referred to by its abbreviation DL-PLGA.

The nanofibres of the bioveil have a nanometric diameter between 500 and 1500 nm and are disposed randomly.

The sheet forming the bioveil has a grammage between 5 and 10 g/m² and is porous, hydrophilic and absorbable.

The applicant of the present utility model has verified the potential in a wide range of biomedical applications, and it is particularly advantageous in promoting cell anchorage, better skin graft take, the improvement of the quality of the healed tissue in patients with second- and third-degree burns, and promoting blood vessel generation.

Namely, the sheet forming the bioveil of the invention is a product that can be implanted on wounds and/or can be implanted by means of surgical methods, being intended for prolonged use as it is completely absorbed during the period of use.

The bioveil is preferably obtained by the known technology of electrospinning, such that the nanofibres have very high surface-to-weight ratio compared with conventional tissues.

The structure of the bioveil provides a biomimetic support for cell growth, improving the autologous cell culture take in skin lesions caused by burns, helping with cell anchorage and proliferation, generating lower culture rejection rates, a reduction of the healing time, and the improvement of quality of the resulting scars in terms of elasticity and shrinkage.

The surface of the bioveil is activated when subjected to low-pressure nitrogen plasma for at least 5 seconds. The activation of the surface of the bioveil improves its handleability and the hydrophilicity of the nanofibre structure, thereby allowing interaction with the culture medium and/or live tissues. Advantageously, the surface-activated bioveil will be more receptive to moisture, losing its tendency to accumulate static electricity.

Moreover, it should be indicated that the bioveil of the present invention is presented in a heat-sealed and sterilised package in which one unit in the form of a sheet, preferably having a size of 100 x 150 mm, is included.

For storage of the bioveil, it must be kept at a temperature of less than 6ºC until the time of use.

The manner of using the bioveil of the present invention on a wound to be treated is described in detail below by way of example.
- Remove the residues of any prior medication and wash the wound and the area surrounding it (with a 0.9% sodium chloride solution [saline solution]).
- Then dry the wound.
- Open the package containing the bioveil in an aseptic manner, take the bioveil out, and dispose of the package.
- Remove the wax-coated cellulose support and arrange the bioveil comprising nanofibres on the wound, with its edges covering the entire surface.
- Ensure close contact between the bioveil and the surface of the wound, avoiding creases being formed, air being trapped, or folds occurring in the bioveil comprising nanofibres.
- Fix the bioveil with surgical glue or suture.
- Apply on the veil comprising nanofibres the keratinocyte culture or graft, covering the affected area.
- Apply dressing to fix the culture.

The bioveil of the invention for tissue regeneration thereby mimics the extracellular matrix of the native tissue and allows better cell anchorage and a temporary scaffold having a controlled duration of up to 90 days to be obtained for healing wounds by means of new dermal tissue growth and formation.

Likewise, the bioveil generates 15% more angiogenesis compared to a sheet comprising bovine collagen. Advantageously, the bioveil is completely absorbed between 30 days and 90 days after its implantation, during which time it generates an excellent take when applied in skin grafts, achieving graft takes of 80%, compared to 20% - 30% offered by products commonly used for these purposes.

### DESCRIPTION OF THE DRAWINGS

To complement the description that is provided below and for the purpose of helping to better understand the features of the invention according to a preferred practical embodiment thereof, a set of drawings is attached as an integral part of said description in which the following is depicted in an illustrative and non-limiting manner:
Figure 1 shows an image of a sheet of the bioveil of the present invention, observed under a scanning electron microscope (SEM) at x3000.
Figure 2 shows the healing status of a wound 15 days after applying the bioveil of the invention and after applying a sheet comprising bovine collagen.
Figure 3 shows the healing status of a wound 30 days after applying the bioveil of the invention and after applying a sheet comprising bovine collagen.
Figure 4 shows the graph representing vascularisation, expressed as blood vessels per mm², generated by the bioveil of the invention and by a sheet comprising collagen two weeks after dermal implantation.
Figure 5 shows the placement of the bioveil of the invention as an intermediate layer in a burn.

### DETAILED DISCLOSURE OF THE TESTS RELATING TO THE INVENTION

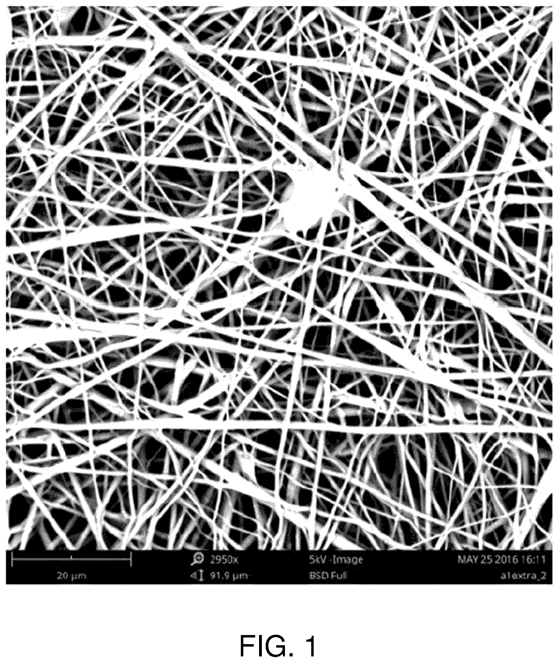

As can be seen in Figure 1, the structure of the bioveil of the invention is in the form of a very porous sheet, and a preferred embodiment consists of a sheet having a thickness of 49 micrometers, made up of fibres that are disposed randomly having mean nanometric diameters of 887 nm and having a biomimetic behaviour for a preferred grammage of 7.7 g/m2, with evident isotropy and maximum tensile strength of 3.40 MPa, with a strain at break of 75.87%.

Experimental tests were performed according to the corresponding laws and regulations (European Convention no. 123 for the protection of vertebrate animals used for experimental and other scientific purposes, Spanish Royal Decree 223/88 European and Spanish, and Ministerial Order 13-10-89 of the Ministry of Agriculture, Food, and Fishing, Protection and Use of Animals in Scientific Research, and the internal regulation on Biosafety and Bioethical Guidelines) for the purpose of verifying the properties of the bioveil of the present invention.

Namely, a study was performed in which the bioveil is subcutaneously implanted in 10 mice for a time period of 30 days, and after histology, it was observed that the bioveil of the invention had been completely degraded.

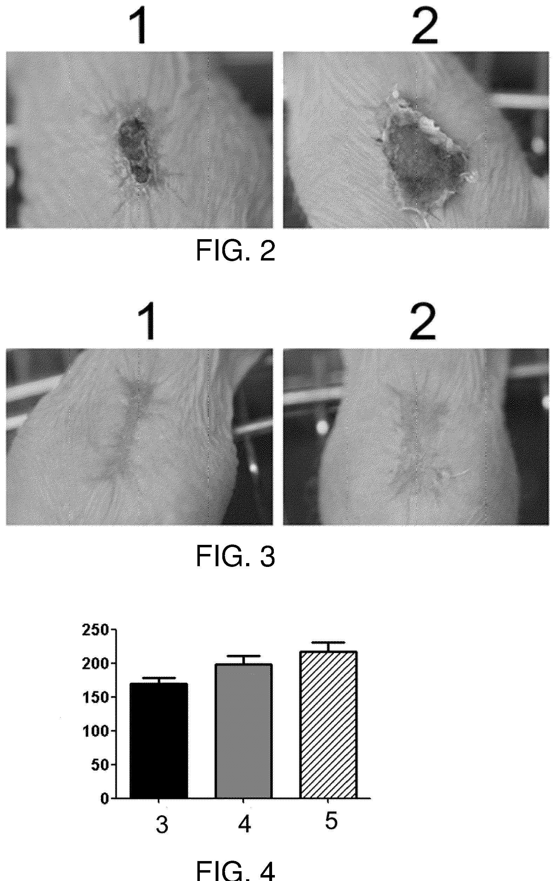

To determine the behaviour of bioveils when they are implanted together with skin grafts, *in vivo* tests were performed. The model used was the xenogeneic transplantation on an open wound bed, using NOD/SCID immunocompromised mice. The test was performed with five mice for each bioveil. Namely, the behaviour of a bioveil according to the features of the present invention and that of a sheet comprising bovine collagen were analysed in the test. Photographs were taken during the test after 15 and 30 days to observe the healing process. Figure 2 shows healing with the use of the sheet comprising bovine collagen (1) and with the use of the bioveil of the invention (2) 15 days after implantation in a mouse.

Figure 3 shows healing with the use of the sheet comprising bovine collagen (1) and with the use of the bioveil of the invention (2) 30 days after implantation in a mouse.

In this sense, the macroscopic viewing of the skin grafted on the bioveil of the invention shows an excellent take of the graft; namely, when disposed on a wound it reduces wound contraction by up to 69%, which generates quality scars, recovering sensitivity and elasticity. However, the sheet comprising bovine collagen provides a less developed scar showing much more contraction (views depicted in (2) of Figures 2 and 3).

The comparative study between bioveils allows concluding that the bioveil of the invention is a better polymer substrate for skin reconstruction than the sheet comprising collagen due to its angiogenic properties, which allow blood vessel generation to be promoted.

This conclusion is observed in Figure 4, where vascularisation, expressed as blood vessels per mm² generated by the bioveils of the invention (5) and by the sheet comprising bovine collagen (4) two weeks after dermal implantation, is depicted on the y-axis.

The control sample (3) used in these comparative studies is likewise depicted in the graph in Figure 4. In that sense, it can be asserted for the bioveil of the invention that blood vessel generation is 15% greater than the generation provided by a sheet comprising bovine collagen and 30% greater than the generation provided by the control sheet.

For implantation of the bioveil in serious burns in which the epidermis of the skin is affected and an autologous graft or culture of keratinocytes will be needed in order to replace the affected layer of skin, the bioveil of the invention acts like an intermediate layer to facilitate the anchorage and take of the coverage.

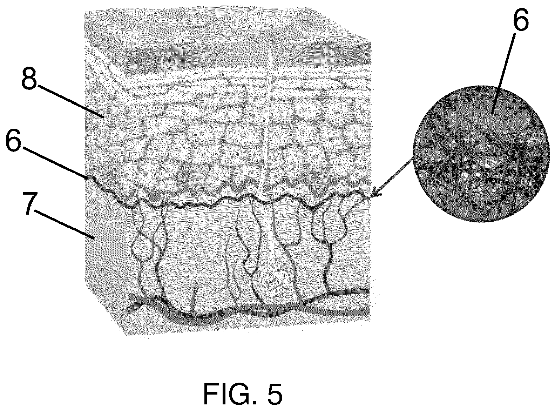

In that sense, Figure 5 depicts the placement of the bioveil of the invention (6) as an intermediate layer in a burn (7) to improve the take of the epidermal graft (8). Complementarily, Figure 5 includes an enlarged view of the bioveil (6) the polymer nanofibres of which are disposed randomly.

Based on the foregoing, it can be asserted that the bioveil object of the present invention facilitates the anchorage and take of autologous grafts, promotes wound healing by generating quality scars and recovering area elasticity and sensitivity, with all these properties having been scientifically proven.

## Claims

1. A bioveil for the treatment of wounds and burns, **characterised in that** it is formed by a sheet comprising tangled polymer nanofibres of 50:50 esterified poly(D,L-lactic-co-glycolic) acid, wherein the nanofibres have a nanometric diameter between 500 and 1500 nm and are disposed randomly, and the sheet has a grammage between 5 and 10 g/m² and is porous, hydrophilic and absorbable so as to promote cell anchorage, better skin graft take, the improvement of the quality of the healed tissue in patients with second- and third-degree burns, and blood vessel generation.

2. The bioveil for the treatment of wounds and burns according to claim 1, **characterised in that** it has a degradation time between 30 and 90 days after being placed on a wound.

3. The bioveil for the treatment of wounds and burns according to claim 1, **characterised in that** its surface is activated when subjected to low-pressure nitrogen plasma for at least 5 seconds.
